Europäisches Patentamt

European Patent Office

Office européen des brevets

(11) Publication number: **0 092 344**
**A1**

## EUROPEAN PATENT APPLICATION

(21) Application number: **83301943.3**

(22) Date of filing: **06.04.83**

(51) Int. Cl.³: **G 01 N 33/54, G 01 N 33/58**

(30) Priority: **15.04.82 GB 8210928**

(43) Date of publication of application: **26.10.83**
**Bulletin 83/43**

(84) Designated Contracting States: **DE FR GB**

(71) Applicant: **AMERSHAM INTERNATIONAL plc, White Lion Road, Amersham Buckinghamshire, HP7 9LL (GB)**

(72) Inventor: **Allen, Gerald John, c/o Amersham International plc White Lion Road, Amersham Buckinghamshire, HP7 9LL (GB)**

(74) Representative: **Pennant, Pyers et al, Stevens, Hewlett & Perkins 5 Quality Court Chancery Lane, London, WC2A 1HZ (GB)**

(54) **Assay method.**

(57) A method of assaying an analyte in a sample uses a specific binding partner for the analyte, a labelled derivative of the analyte, and discrete solid particles having thereon a material which is not member of the specific binding pair but which controls the extent of binding of the labelled derivative, the solid particles having the propety of modifying the signal produced by labelled derivate bound to them. The label is preferably a fluorescent molecule and the solid particles are preferably of carbon, either coated with albumin or carrying a specific receptor for the specific binding partner for the analyte. When a mixture of the sample, the solid particles, the labelled analyte derivative and the specific binding partner is incubated, part of the labelled derivative becomes bound to the solid particles, and the signal emitted by the homogeneous mixture can be used to assay the analyte.

0092344

PP/JR/1464

## ASSAY METHOD

This invention relates to an assay method for determining the presence in a sample of an analyte which is a member of a specific binding pair consisting of ligand and its specific receptor. Such assay methods are well known and have been widely used for many years. Often the analyte is a material found in a human or animal body and the sample is, or is derived from, a body fluid.

Such assay methods employ a signal-producing system consisting of one or more members at least one of which is associated with a member of the specific binding pair to form a signal conjugate. For example, in a well-known assay method for vitamin B12, the analyte is vitamin B12, the specific receptor is Intrinsic factor and the signal-producing system is radioactive Cobalt-57, associated with the analyte as vitamin B12 - $^{57}$Co which is the signal conjugate. Typical signal-producing systems are radioactive atoms, fluorescing molecules and enzymes.

In these assay methods, part of the signal conjugate becomes bound to the specific receptor while the remainder of the signal conjugate does not become so bound. The proportion of signal conjugate so bound depends on the concentration of analyte in the sample. Measurement of the proportion of the signal conjugate so bound, or of the proportion not bound, enables the concentration of analyte in the sample to be determined.

In heterogeneous assays, the signal conjugate bound to the specific receptor is separated from the signal conjugate not so bound. Such separation is usually necessary in radioassays, because the signal emitted by the signal-producing systems (radioactive atoms) is usually the same, whether or not the signal conjugate is bound. But the separation process is tedious, time consuming, difficult to automate, and a source of inaccuracy.

In homogeneous assays, the signal is measured without prior separation of the bound and not-bound portions of the signal conjugate. This has the advantage of avoiding the aforesaid tedious separation step. But it is only possible when the signal produced by the bound portion of the signal conjugate can be distinguished from the signal produced by the not-bound portion. Such distinction can often be made, particularly when the signal-producing system is a fluorescing molecule or an enzyme. Homogeneous assays are now in many cases a viable alternative to heterogeneous assays. This invention is concerned only with homogeneous assays, and not with heterogeneous assays.

U.S. Patent 4275149 relates to homogeneous assays, and is concerned with distinguishing the signal produced by the bound portion of the signal conjugate from the signal produced by the not-bound portion. The patent uses "particle conjugates", that is to say dispersed particles which carry one of the members of the specific binding pair. A portion of a signal conjugate becomes bound to the particles by the specific binding reaction. The particles modify the environment of the portion of the signal conjugate bound to them, for example by using particles which cause a different surface pH from the aqueous medium or by using hydrophobic particles, and so modify the signal emitted by the bound portion of the signal conjugate.

The method of U.S. Patent 4275149 suffers from some disadvantages. Thus, a different particle conjugate needs to be used for each different analyte assayed; it would be convenient to be able to use the same particles for several different assays. Also, no effective means are described for preventing signal conjugate from becoming bound to the particles by simple physical adsorption; if this happened to any significant extent, the assay would be invalidated.

U.S. Patent 4256834 relates to a system which is similar except that the signal-producing system is necessarily chromophoric or fluorophoric and not an enzyme. Part of the signal conjugate becomes bound to a particle conjugate by a specific binding reaction, but no attempt is made to alter the signal emitted by this bound signal conjugate. The remainder of the signal conjugate is physically adsorbed by other dispersed particles called "signal repressors" and the signal emitted by this remainder is altered or suppressed.

The method of U.S. Patent 4256834 suffers from the same disadvantage as that of U.S. Patent 4275149, namely that a different particle conjugate needs to be used for each different analyte assayed. Also, the method uses two different sets of dispersed particles, which complicates the system and reduces accuracy.

U.S. Patent 4318707 relates to a system which is also similar to that of U.S. 4275149 except that the signal producing system is fluorescent or chemiluminescent. Particle conjugates are provided comprising one member of the specific binding pair bound to light absorbing particles, particularly of carbon. Part of a signal conjugate becomes bound to the particles by the specific binding reaction. The particles adsorb, and thus reduce or eliminate, the signal produced by that part of the signal conjugate. The method of this patent suffers from the same disadvantage as the other two patents mentioned, namely that a different particle conjugate needs to be used for each different analyte assayed.

The present invention overcomes this disadvantage, and enables the same particle conjugate to be used for assaying a variety of different analytes.

According to the present invention, there is provided an assay method for determining the presence in a sample of an analyte which is a member of a specific binding pair, which method employs;

a) a specific binding partner for the analyte, being the other member of the specific binding pair;

b) a signal-producing system consisting of one or more members of which at least one is conjugated to the analyte to form a signal conjugate;

c) discrete dispersable solid particles having thereon a material which is not a member of the specific binding pair but which controls the extent to which the signal conjugate becomes bound to the solid particles, the solid particles having the property of modifying the signal produced by that part of the signal-producing system bound thereto;

said method comprising combining in an aqueous assay medium the sample, the signal conjugate, the specific binding partner for the analyte, and any remaining members of the signal producing system, and incubating the resulting mixture;

providing the solid particles substantially uniformly dispersed in the aqueous medium before, during or after said incubation, whereby a proportion of the signal conjugate becomes bound to the solid particles, the said proportion being related to the amount of analyte in the sample; and

determining the level of the signal in the aqueous assay medium compared to an assay medium containing a sample having a known concentration of analyte.

The solid particles can be added at the beginning of the assay method or at any time during the establishment of equilibrium between the specific binding partners, but are preferably added when equilibrium has been attained. Depending on the nature of the material on the particles, there may become bound to the particles, either the portion of the lable conjugate bound to the specific binding partner, or the portion of the label conjugate not so bound.

The nature of the analyte is not critical. The analyte may conveniently be a hapten or antigen and the specific binding partner its antibody. Alternatively, the analyte could be the antibody, in which case the specific binding partner would be its antigen. Alternatively, the two members of the specific binding pair (the analyte and its specific binding partner) may bind together by other than an immune reaction. The analyte may for example be a hormone, a biochemical messenger, a steroid, a drug, a drug metabolite, a protein or polypeptide, a catecholamine, a vitamin, a tumour antigen, a toxin, an alkaloid or a mono-, di- or polysaccharide.

The signal-producing system involves a catalyst, particularly an enzyme, or a chromophore, or a chemiluminescent or fluorescent material. When the signal-producing system involves an enzyme, it will generally consist of two or more members (e.g. enzyme and substrate) of which one will be conjugated to the analyte to form the signal conjugate. When the signal-producing system involves a fluorescent material, it will generally consist of one member conjugated to the analyte to form the signal conjugate. Representative fluorescent materials include fluorescein, tetramethylrhodamine, umbelliferone and rare earth metal chelates. Techniques for conjugating such materials to the analyte are well-known and will not be described here.

Discrete dispersable solid particles are used which have the property of modifying the signal produced by that part of the signal-producing system bound to them.  This modification of the signal may be achieved in various ways.  For example, the environment of the particles may be arranged to be different from the environment of the surrounding aqueous medium in such a way, e.g. as to pH, as to modify the properties of the signal conjugate.  Alternately, when the signal is emitted electromagnetic radiation, the particles may be opaque at the emitted wavelength;  if the particle is porous or reticular, this may result in almost complete suppression of the emitted signal.  For example:-

a)  Carbon is opaque at all visible and UV wavelengths.  Carbon particles such as activated charcoal have a large surface area and are porous.  Such particles are highly effective to suppress radiation emitted by materials bound to them.  Particles substantially or wholly of carbon are preferred for use in this invention.

b)  Other particulate materials with high surface area include polysaccharides, dextran, cellulose, starch, polyacrylamides, polystyrene, polymethacrylates and porous glasses.  If such materials are not opaque at the desired wavelength, they can be made so by means of a dye.  Thus, rhodamine absorbs light at the fluorescence wavelength of fluorescein, and can be used to opacify particles by absorption.  It is not necessary that the particles should be completely opaque at the relevant wavelength.  But they must be sufficiently opaque to substantially modify the signal generated by materials bound to them.

The particles have thereon a material which controls the extent to which the signal conjugate becomes bound thereto.  Particularly when carbon particles are used, this is an important feature, for most components of the assay are strongly adsorbed and bound by carbon.  The material used is preferably a proteinaceous or other molecular sieve type, such as plasma, blood fractions, albumin or dextran. It may form a thin coating, one molecule or a few molecules thick, on the surface of the particle. It may be reactive with a component of the assay system; for example, when the specific binding partner of the analyte is antigenic, it may be an antibody to the specific binding partner.

Alternatively, it may simply act as a physical sieve to permit molecules below a certain size to become bound to the underlying particle. Solid particles with a coating which does not include either the analyte or its specific binding partner have the advantage that they can be used for the assay of a variety of different analytes.

The use of coated charcoal particles is widespread in the field of radio-assays. See for example U.S. Patent 3442819. But in radio-assays the charcoal is used to permit physical separation of the bound portion of the signal conjugate from the not-bound portion. By contrast, in the method of the present invention, no such physical separation is effected, the charcoal being used to suppress the signal emitted by the bound portion of the signal conjugate.

There follow descriptions of two systems envisaged according to the invention. The analyte and its specific binding partner are called analyte and antibody, although as noted above they could be any specific binding pair. The member of the signal-producing system used is called for simplicity, the label.

The solution reactants are the sample being assayed, labelled analyte, and antibody. When these reactants are mixed, part of the labelled analyte, the proportion depending on the amount of analyte in the sample, becomes bound to the antibody.

A. In this system, the solid particles are coated with albumin or other molecular sieve. These adsorb molecules below a certain size, including the labelled analyte, but exclude the labelled analyte/antibody complex.

Albumin is very suitable in cases where the labelled analyte has a molecular weight up to about 3000. For larger molecules, alternative molecular sieves are required which can achieve the discrimination between labelled analytes and the labelled analyte/antibody complex. The same coated particles can be used for assaying a variety of different analytes.

B. In this system, the solid particles carry a substance, herein designated "second antibody", which is a specific receptor for the antibody. Essentially all of the labelled analyte/antibody complex becomes bound to the particles.

Second antibodies of the kind referred to are readily available. Moreover, they act as specific receptors to a variety of different antibodies. Thus, this system also has the advantage that the same particles (carrying second antibody) can be used in assays for a variety of different analytes.

Assays according to this invention may be performed under conditions of time, temperature, pH, proportions and order of addition of reactants, which are conventional.

These assays are generally performed using a plurality of tubes, some containing standard amounts of analyte and others containing unknown samples, using the standards to generate a graph of measurement against analyte concentration, and using the graph to determine the concentration of analyte in the unknown sample. It is essential that the amount of solid particles used be accurately the same from tube to tube.

It is also essential that the solid particles be substantially uniformly distributed throughout the aqueous medium throughout the duration of the measurement.

Fluorescent materials are ones which adsorb light (or other electromagnetic radiation) of a particular excitation wavelength, undergo internal molecular changes, and then emit light at a somewhat longer emission wavelength. The technique of fluorimetry involves shining preferably monochromatic light of excitation wavelength at the assay mixture and observing light emitted at the emission frequency.

The concentration of solid particles used must clearly be sufficient to effect the desired physical or chemical change in the aqueous medium during the incubation time of the assay. Fluorimetry requires that the aqueous solution under test be substantially transparent. If the concentration of solid particles is so high that the aqueous solution under test is substantially opaque, then accuracy and sensitivity are reduced. It is preferred not to use a large excess of solid particles. Typically, the concentration of solid particles used will give a 25% to 75% transmission under the assay conditions.

The following Example illustrates the invention. The system is of Type A of those noted earlier.

### Fluorescence Assay of Phenytoin Amine

Antiserum to a phenytoin-amine derivative was diluted in 0.02M tris buffer-0.15M sodium chloride at pH 8.2 containing 0.1% ($^W$/v) bovine serum albumin.

Phenytoin-fluorescein label was prepared by reacting 10mg of an amine derivative of phenytoin with 12.3mg of fluorescein isothiocyanate in a buffer composed of pyridine: triethylamine: water (9: 0.1: 1.5 v/v/v) overnight at 4°C. 0.5M tris buffer -0.15M sodium chloride at pH 8.2 was then added, and the mixture was incubated for one hour at room temperature and then diluted in 0.02M tris buffer -0.15M sodium chloride at pH 8.2 containing 0.1% ($^W$/v) bovine serum albumin.

The solid particles were of albumin coated charcoal as used in commercial radioimmunoassay kits. The albumin coating acts as a molecular sieve to accept phenytoin-fluorescein label but to exclude antiserum and complexes thereof. The coated particles were ground and then suspended in water, and the fraction was used that remained in suspension for 12 hours.

The assay protocol was to mix 50$\mu$l of a standard solution of phenytoin amine in serum with 200$\mu$l label and 100$\mu$l antiserum. After incubation for 15 minutes at room temperature, 4 ml of 0.02M tris buffer - 015M sodium chloride at pH 8.2 containing the albumin coated charcoal particles was added. The solid particles reduced transmission of the mixture in the 1cm cell used for assay purposes by 50%. The mixture was incubated for a further 15 minutes. The fluorescence of the solution was quantified in a 1cm cell using excitation light of 490 nm and measuring the emitted light of 520 nm. The following figures were obtained.

| Concentration of Phenytoin Amine ($\mu$g/ml) | Fluorescence (Arbitrary Units) |
|---|---|
| 0 | 19000 |
| 3.125 | 10800 |
| 6.25 | 9150 |
| 12.5 | 8150 |
| 25.0 | 7000 |
| 50.0 | 6150 |
| 100.0 | 5450 |

C L A I M S

1. An assay method for determining the presence in a sample of an analyte which is a member of a specific binding pair, which method employs;

a) a specific binding partner for the analyte, being the other member of the specific binding pair;

b) a signal-producing system consisting of one or more members of which at least one is conjugated to the analyte to form a signal conjugate;

c) discrete dispersable solid particles having thereon a material which is not a member of the specific binding pair but which controls the extent to which the signal conjugate becomes bound to the solid particles, the solid particles having the property of modifying the signal produced by that part of the signal-producing system bound thereto;

said method comprising combining in an aqueous assay medium the sample, the signal conjugate, the specific binding partner for the analyte, and any remaining members of the signal producing system, and incubating the resulting mixture;

providing the solid particles substantially uniformly dispersed in the aqueous medium before, during or after said incubation, whereby a proportion of the signal conjugate becomes bound to the solid particles, the said proportion being related to the amount of analyte in the sample; and

determining the level of the signal in the aqueous assay medium compared to an assay medium containing a sample having a known concentration of analyte.

2. A method as claimed in claim 1, wherein the analyte is a hapten or an antigen and the specific binding partner is its antibody.

3. A method as claimed in claim 1 or claim 2,
wherein the solid particles are porous or reticular.

4. A method as claimed in any one of claims 1 to 3,
wherein the signal is emitted electromagnetic radiation
and the solid particles are opaque to the signal.

5. A method as claimed in any one of claims 1 to 4,
wherein the solid particles are substantially of carbon.

6. A method as claimed in any one of claims 1 to 5,
wherein the solid particles are coated with a
molecular sieve which adsorbs the analyte and the labelled
analyte but excludes the analyte bound to its specific
binding partner.

7. A method as claimed in claim 6,
wherein the molecular sieve is albumin.

8. A method as claimed in any one of claims 1 to 5,
wherein the solid particles carry a substance which is
a specific receptor for the specific binding partner of
the analyte.

9. A method as claimed in any one of claims 1 to 8,
wherein the signal-producing system is a fluorescent
material conjugated to the analyte.

10. A method as claimed in claim 9,
wherein the concentration of solid particles used gives
an assay mixture having a 25% to 75% transmission under
assay conditions.

0092344

European Patent Office

**EUROPEAN SEARCH REPORT**

Application number

EP 83 30 1943

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | EP-A-0 017 908 (SYVA COMPANY) * Abstract; claims 1-20; page 19 * | 1-10 | G 01 N 33/54 G 01 N 33/58 |
| P,A | EP-A-0 063 852 (SYVA COMPANY) * Abstract; claims 1-10 * | 1-10 | |
| A | US-A-4 048 298 (G. NISWENDER) * Abstract * | 1,8 | |
| A | US-A-3 442 819 (V. HERBERT) * Abstract * | 6-8 | |

TECHNICAL FIELDS SEARCHED (Int. Cl. 3)

G 01 N

The present search report has been drawn up for all claims

| Place of search THE HAGUE | Date of completion of the search 22-07-1983 | Examiner OSBORNE H.H. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82